(19) Europäisches Patentamt
European Patent Office
Office européen des brevets

(11) EP 4 767 976 A1

(12) **EUROPEAN PATENT APPLICATION**

(43) Date of publication:
**01.07.2026 Bulletin 2026/27**

(21) Application number: **26150069.8**

(22) Date of filing: **02.01.2026**

(51) International Patent Classification (IPC):
**A61B 18/14** (2006.01)    **A61B 17/32** (2006.01)
**A61B 18/12** (2006.01)

(52) Cooperative Patent Classification (CPC):
**A61B 18/1445; A61B 17/320092;** A61B 18/1206;
A61B 2017/320093; A61B 2017/320095;
A61B 2018/00607; A61B 2018/00875;
A61B 2018/00994

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **31.12.2024 US 202463740944 P
26.09.2025 US 202519341037
26.09.2025 US 202519341047
26.09.2025 US 202519341060
26.09.2025 US 202519341070**

(71) Applicant: **Cilag GmbH International
6300 Zug (CH)**

(72) Inventors:
• **WEISENBURGH, II,, William B.**
**Cincinnati, 45242 (US)**
• **SAMUEL,, Jonathan**
**Cincinnati, 45242 (US)**
• **JAYME,, Madeleine**
**Cincinnati, 45242 (US)**
• **WIENER,, Eitan**
**Cincinnati, 45242 (US)**
• **LUCAS,, Guion**
**Cincinnati, 45242 (US)**
• **GONENC,, Berk**
**Cincinnati, 45242 (US)**

(74) Representative: **Carpmaels & Ransford LLP
One Southampton Row
London WC1B 5HA (GB)**

(54) **TECHNOLOGIES FOR CONTROLLING THERAPEUTIC AND SUBTHERAPEUTIC ACTIVATION OF ENERGY-BASED SURGICAL INSTRUMENTS**

(57) A control method for an energy-based surgical instrument includes activating an energy control signal at a subtherapeutic level, measuring a system response at the subtherapeutic level, adjusting one or more parameters based on the system response, and then activating an energy control signal at a therapeutic level according to the determined parameters. The subtherapeutic and therapeutic signals may be ultrasound or radio frequency (RF) energy and/or a combination of ultrasound and RF energy. Controlled parameters may include mode, cycle time, power (e.g., ultrasound tip displacement), or crest factor. Other embodiments are described and claimed.

EP 4 767 976 A1

FIG. 8

**Description**

**CROSS-REFERENCE TO RELATED APPLICATION**

This application claims the benefit of and priority to U.S. Patent Application No. 63/740,944, entitled "TECHNOLOGIES FOR THERAPEUTIC AND SUBTHERAPEUTIC CONTROL OF ENERGY-BASED SURGICAL INSTRUMENTS," which was filed on December 31, 2024, and which is incorporated herein by reference in its entirety.

**TECHNICAL FIELD**

The present disclosure relates generally to energy-based surgical instruments and, more particularly, to harmonic and/or electrosurgical surgical instruments.

**BACKGROUND**

Energy-based surgical instruments are finding increasingly widespread applications in surgical procedures by virtue of their unique performance characteristics. Depending upon specific device configurations and operational parameters, energy-based surgical instruments can provide both transection of tissue and hemostasis of the tissue by coagulation, which may reduce or otherwise minimize patient trauma. Depending on the particular application, energy-based surgical instruments may utilize different surgical technologies including, for example, ultrasonic and/or electro-surgical (e.g., radio frequency (RF)) technologies.

A typical ultrasonic surgical instrument may include a handpiece containing an ultrasonic transducer and an elongated shaft assembly having a distally mounted end effector to effect the cutting and sealing of tissue. For example, the end effector may include a jaw assembly having an ultrasonic blade and a clamp arm, which may include a non-stick tissue pad or similar bed to receive the ultrasonic blade. In some cases, the elongated shaft assembly may be permanently affixed to the handpiece. In other cases, the elongated shaft assembly may be detachable from the handpiece, as in the case of a disposable shaft assembly or a shaft assembly that is interchangeable between different handpieces. In use, the end effector transmits ultrasonic energy to tissue brought into contact with the ultrasonic blade of the end effector to realize the cutting and sealing action. Such ultrasonic surgical devices may be configured for open surgical use, laparoscopic, and/or endoscopic surgical procedures including robotic-assisted procedures.

Ultrasonic energy cuts and coagulates tissue using temperatures lower than those used in electro-surgical procedures. Vibrating at high frequencies (e.g., 55,500 times per second), the ultrasonic blade denatures protein in the tissue to form a sticky coagulum. Pressure exerted on tissue by the ultrasonic blade surface collapses blood vessels and allows the coagulum to form a hemostatic seal. A surgeon can control the cutting speed and coagulation by the force applied to the tissue by the end effector, the time over which the force is applied, and the selected excursion level of the end effector.

In electro-surgical instruments, one or more electrodes are incorporated into the end effector and configured to apply therapeutic electrical current to the patient's tissue to create a hemostatic seal. In electro-surgical instruments that do not include a harmonic mode (i.e., do not include a harmonic blade), the end effector may be embodied as two clamp arms or jaws. In such embodiments, the electro-surgical instrument may include a separate mechanical knife or blade for cutting the tissue after the creation of the hemostatic seal, which may be incorporated into the elongated shaft attached to the end effector. In bi-polar embodiments, an active electrode may be attached to one of the clamp arms of the end effector and configured to introduce an electrical current into the tissue, which is received by a return electrode attached to the other clamp arm of the end effector (or as the blade itself in embodiments including a harmonic mode). Conversely, in mono-polar embodiments, the return electrode (e.g., a "grounding pad") may be separate from the electro-surgical instrument and located on a different part of the body of the patient. In some embodiments, the electro-surgical instrument may also be configured to apply a sub-therapeutic electrical current to the patient's tissue, which may be used for sensing purposes (e.g., measuring tissue impedance).

Electro-surgery forms hemostatic seals by generating heat in the tissue via the introduced electrical energy, which is embodied as radio frequency ("RF") energy. The particular frequency employed can vary based on the intended use of the electro-surgical instrument within the range of about 100kHz to 1 MHz, although higher frequencies can be employed in some embodiments. Additionally, sub-therapeutic frequencies may be used in some situations for purposes other than hemostatic sealing, such as performing various electrical measurements on the tissue.

It should be appreciated that some energy-based surgical instruments may employ dual or multi-modal technologies for the transection and/or hemostasis of patient tissue. For example, in some cases, an energy-based surgical instrument may include both ultrasonic and electro-surgical capabilities (e.g., by utilizing the ultrasonic blade as an electrode for the electro-surgery mode), which increases the surgical options provided by the surgical instrument to the surgeon.

# EP 4 767 976 A1

## SUMMARY

According to an aspect of the present disclosure, a method for controlling a surgical instrument includes activating, by a control element, a first energy signal at a subtherapeutic level, wherein the first energy signal is output by a surgical generator coupled to the surgical instrument, and wherein the surgical instrument comprises an end effector configured to deliver energy to tissue of a patient; measuring, by the control element, a system response in response to activating the first energy signal; determining, by the control element, an activation parameter based on the system response; and activating, by the control element, a second energy signal at a therapeutic level according to the activation parameter, wherein the second energy signal is output by the surgical generator.

In some embodiments, the first energy signal comprises an ultrasound signal or a radio frequency (RF) signal; and the second energy signal comprises an ultrasound signal or a radio frequency (RF) signal. In some embodiments, the system response comprises impedance, rate of change of impedance, or acoustic impedance. In some embodiments, the activation parameter comprises power, current, or duration.

According to another aspect, a method for controlling a surgical instrument includes determining, by a control element of the surgical instrument, an activation mode for the surgical instrument, wherein the surgical instrument comprises an end effector having a first electrode configured to deliver radio frequency (RF) energy to tissue of a patient; determining, by the control element, a waveshape for an energy signal based on the activation mode; programming, by the control element, an electrosurgical generator coupled to the surgical instrument with the waveshape in response to determining the waveshape; and delivering, by the electrosurgical generator, the energy signal to the electrode of the end effector in response to programming the electrosurgical generator.

In some embodiments, the activation mode comprises a cutting mode, a coagulation mode, or blended mode. In some embodiments, determining the waveshape includes determining a crest factor for the waveshape based on the activation mode.

In some embodiments, determining the waveshape includes determining a first waveshape having a first crest factor corresponding to a sinusoidal waveshape when the activation mode is the cutting mode. In some embodiments, determining the waveshape comprises includes a second waveshape having a second crest factor larger than the first crest factor when the activation mode is the coagulation mode. In some embodiments, the second waveshape comprises a pulse train of damped sinusoidal waves.

In some embodiments, determining the waveshape includes determining a third waveshape having a third crest factor between the first crest factor and the second crest factor when the activation mode is the blended mode. In some embodiments, the third waveshape comprises a pulse train of damped sinusoidal waves blended with an undamped sinusoidal wave. In some embodiments, the third waveshape comprises a sinusoidal wave modulated with a second, lower frequency sinusoidal wave.

In some embodiments, delivering the energy signal includes generating an analog output signal for the energy signal with direct digital synthesis. In some embodiments, programming includes storing digital values indicative of the waveshape in a memory of the electrosurgical generator.

In some embodiments, the method further includes determining, by the control element, an updated waveshape for the energy signal based on an electrical property of tissue sensed by the end effector; and updating, by the control element, programming of the electrosurgical generator with the updated waveshape. In some embodiments, determining the updated waveshape comprises increasing a crest factor of the waveshape from a predetermined crest factor associated with a sinusoidal waveshape.

According to another aspect, a method for controlling a surgical instrument includes performing, by the surgical instrument, a radio frequency (RF) energy cycle with a first electrode of an end effector of the surgical instrument while pressure is applied in a jaw of the surgical instrument; relieving pressure in the jaw of the surgical instrument after performing the RF energy cycle; measuring, by the control element, a first acoustic impedance of the surgical instrument with a subtherapeutic ultrasound signal using an ultrasonic blade of the end effector after relieving the pressure in the jaw of the surgical instrument; determining, by the control element, whether tissue is stuck to the jaw of surgical instrument based on the first acoustic impedance; and activating, by the control element, an automatic ultrasonic energy release cycle with the ultrasonic blade of the surgical instrument in response to determining that tissue is stuck to the jaw of the surgical instrument.

In some embodiments, the method further includes measuring, by the control element, a baseline acoustic impedance of the surgical instrument with the subtherapeutic ultrasound signal; wherein determining whether the tissue is stuck includes comparing the first acoustic impedance to the baseline acoustic impedance. In some embodiments, determining whether the tissue is stuck further includes determining whether a closure switch of the surgical instrument moves to an open position.

In some embodiments, determining whether the tissue is stuck includes determining whether the tissue is stuck based on an absolute value of the first acoustic impedance. In some embodiments, the method further includes measuring, by the control element, acoustic impedance of the surgical instrument with the subtherapeutic ultrasound signal during the RF energy cycle or during a device idle time.

According to another aspect, a system for controlling a surgical instrument includes a surgical instrument, a surgical generator coupled to the surgical instrument, and a control element. The surgical instrument includes an end effector configured to deliver energy to tissue of a patient. The control element is configured to activate a first energy signal at a subtherapeutic level, wherein the first energy signal is output by the surgical generator; measure a system response in response to activation of the first energy signal; determine an activation parameter based on the system response; and activate a second energy signal at a therapeutic level according to the activation parameter, wherein the second energy signal is output by the surgical generator.

In some embodiments, the first energy signal comprises an ultrasound signal or a radio frequency (RF) signal; and the second energy signal comprises an ultrasound signal or a radio frequency (RF) signal. In some embodiments, the system response comprises impedance, rate of change of impedance, or acoustic impedance. In some embodiments, the activation parameter comprises power, current, or duration.

According to another aspect, a system for controlling a surgical instrument includes a surgical instrument, an electrosurgical generator coupled to the surgical instrument, and a control element. The surgical instrument includes an end effector having a first electrode configured to deliver radio frequency (RF) energy to tissue of a patient. The control element is configured to determine an activation mode for the surgical instrument, determine a waveshape for an energy signal based on the activation mode, and program the electrosurgical generator with the waveshape in response to a determination of the waveshape. The electrosurgical generator is configured to deliver the energy signal to the electrode of the end effector in response to programming of the electrosurgical generator.

In some embodiments, the activation mode comprises a cutting mode, a coagulation mode, or blended mode. In some embodiments, to determine the waveshape includes to determine a crest factor for the waveshape based on the activation mode.

In some embodiments, to determine the waveshape includes to determine a first waveshape having a first crest factor that corresponds to a sinusoidal waveshape when the activation mode is the cutting mode. In some embodiments, to determine the waveshape includes to determine a second waveshape that has a second crest factor larger than the first crest factor when the activation mode is the coagulation mode. In some embodiments, the second waveshape comprises a pulse train of damped sinusoidal waves.

In some embodiments, to determine the waveshape includes to determine a third waveshape that has a third crest factor between the first crest factor and the second crest factor when the activation mode is the blended mode. In some embodiments, the third waveshape comprises a pulse train of damped sinusoidal waves blended with an undamped sinusoidal wave. In some embodiments, the third waveshape comprises a sinusoidal wave modulated with a second, lower frequency sinusoidal wave.

In some embodiments, to deliver the energy signal includes to generate an analog output signal for the energy signal with direct digital synthesis. In some embodiments, to program the electrosurgical generator includes to store digital values indicative of the waveshape in a memory of the electrosurgical generator.

In some embodiments, the control element is further configured to determine an updated waveshape for the energy signal based on an electrical property of tissue sensed by the end effector; and update programming of the electrosurgical generator with the updated waveshape. In some embodiments, to determine the updated waveshape includes to increase a crest factor of the waveshape from a predetermined crest factor associated with a sinusoidal waveshape.

According to another aspect, a system for controlling a surgical instrument includes a surgical instrument and a control element. The surgical instrument includes an end effector comprising a jaw having a first electrode configured to deliver radio frequency (RF) energy to tissue of a patient and an ultrasonic blade configured to deliver ultrasonic energy to the tissue of the patient. The control element is configured to perform an RF energy cycle with the first electrode while pressure is applied in the jaw of the surgical instrument; relieve pressure in the jaw of the surgical instrument after performance of the RF energy cycle; measure a first acoustic impedance of the surgical instrument with a subtherapeutic ultrasound signal using the ultrasonic blade after relief of the pressure in the jaw of the surgical instrument; determine whether tissue is stuck to the jaw of surgical instrument based on the first acoustic impedance; and activate an automatic ultrasonic energy release cycle with the ultrasonic blade of the surgical instrument in response to a determination that tissue is stuck to the jaw of the surgical instrument.

In some embodiments, the control element is further configured to measure a baseline acoustic impedance of the surgical instrument with the subtherapeutic ultrasound signal. To determine whether the tissue is stuck includes to compare the first acoustic impedance to the baseline acoustic impedance. In some embodiments, to determine whether the tissue is stuck further includes to determine whether a closure switch of the surgical instrument moves to an open position.

In some embodiments, to determine whether the tissue is stuck includes to determine whether the tissue is stuck based on an absolute value of the first acoustic impedance. In some embodiments, the control element is further configured to measure acoustic impedance of the surgical instrument with the subtherapeutic ultrasound signal during the RF energy cycle or during a device idle time.

## BRIEF DESCRIPTION OF THE DRAWINGS

The detailed description particularly refers to the following figures, in which:

FIG. 1 is a simplified diagram of an embodiment of a system for performing an energy-based surgical procedure;
FIG. 2 is a perspective view of an embodiment of an energy-based surgical instrument of the system of FIG. 1;
FIG. 3 is a side elevation view of a jaw assembly of an end effector of the surgical instrument of FIG. 2 including an ultrasonic blade and in an open state;
FIG. 4 is a side elevation view of the jaw assembly of the end effector of the surgical instrument of FIG. 2 including an ultrasonic blade and in a closed state;
FIG. 5A is a perspective view of another embodiment of the end effector of the surgical instrument of FIG. 2 including an electrode on a lower jaw clamp of the jaw assembly;
FIG. 5B is a perspective view of another embodiment of the end effector of the surgical instrument of FIG. 2 including two jaw clamps, each having an electrode attached thereto;
FIG. 6 is an exploded view of the surgical instrument of FIG. 2;
FIG. 7 is a block diagram of a control circuit of the surgical instrument of FIG. 2;
FIG. 8 is a simplified flow diagram of at least one method for controlling an energy-based surgical instrument;
FIG. 9 is a simplified flow diagram of at least one method for controlling a radio frequency (RF) or combined energy-based surgical instrument;
FIG. 10 is a schematic diagram illustrating output of the surgical instrument according to the method of FIG. 9;
FIG. 11 is a simplified flow diagram of at least one method for controlling a combined energy-based surgical instrument with automatic ultrasonic release;
FIG. 12 is a chart illustrating measured impedance versus force for one illustrative embodiment of a surgical instrument that may be used with the method of FIG. 11; and
FIG. 13 is a chart illustrating operation of the surgical instrument according to the method of FIG. 11.

## DETAILED DESCRIPTION OF THE DRAWINGS

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Terms representing anatomical references, such as anterior, posterior, medial, lateral, superior, inferior, distal, proximal, et cetera, may be used throughout the specification in reference to the surgical instruments described herein as well as in reference to the patient's natural anatomy. Such terms have well-understood meanings in both the study of anatomy and the field of surgery. Use of such anatomical reference terms in the written description and claims is intended to be consistent with their well-understood meanings unless noted otherwise.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

The disclosed embodiments may be implemented, in some cases, in hardware, firmware, software, or any combination thereof. The disclosed embodiments may also be implemented as instructions carried by or stored on a transitory or non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures.

Additionally, the inclusion of a structural or method feature in a particular figure is not meant to imply that such feature is required in all embodiments and, in some embodiments, may not be included or may be combined with other features.

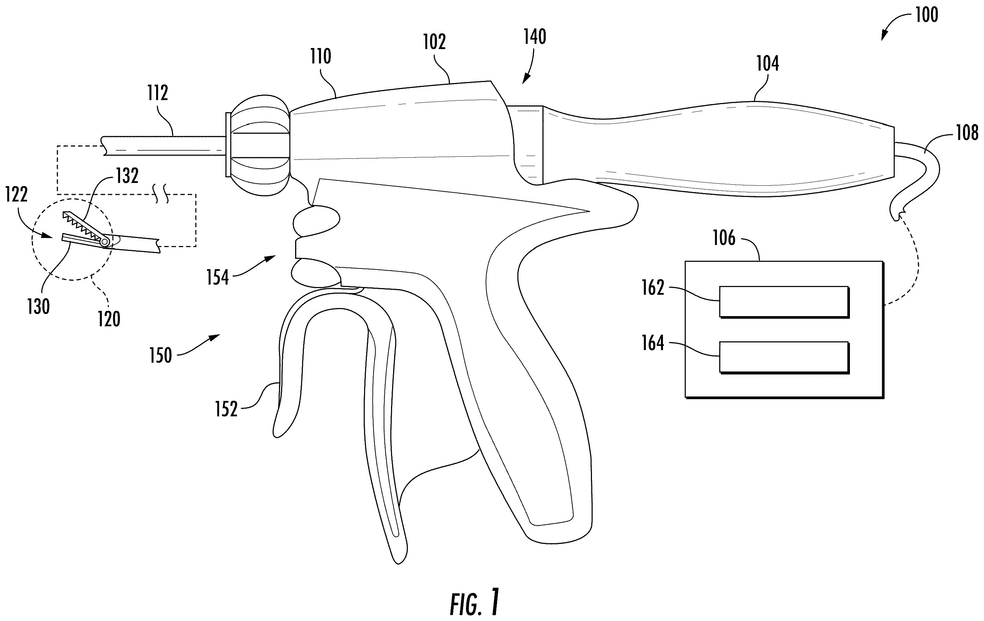

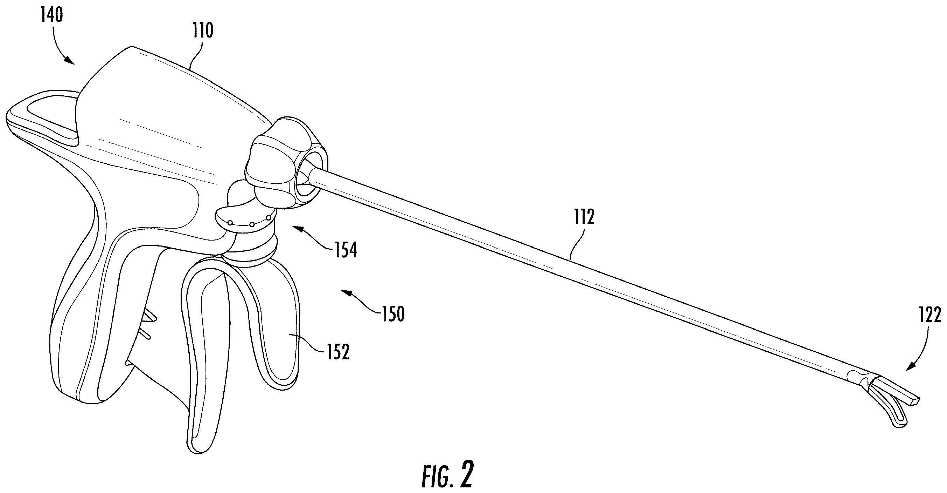

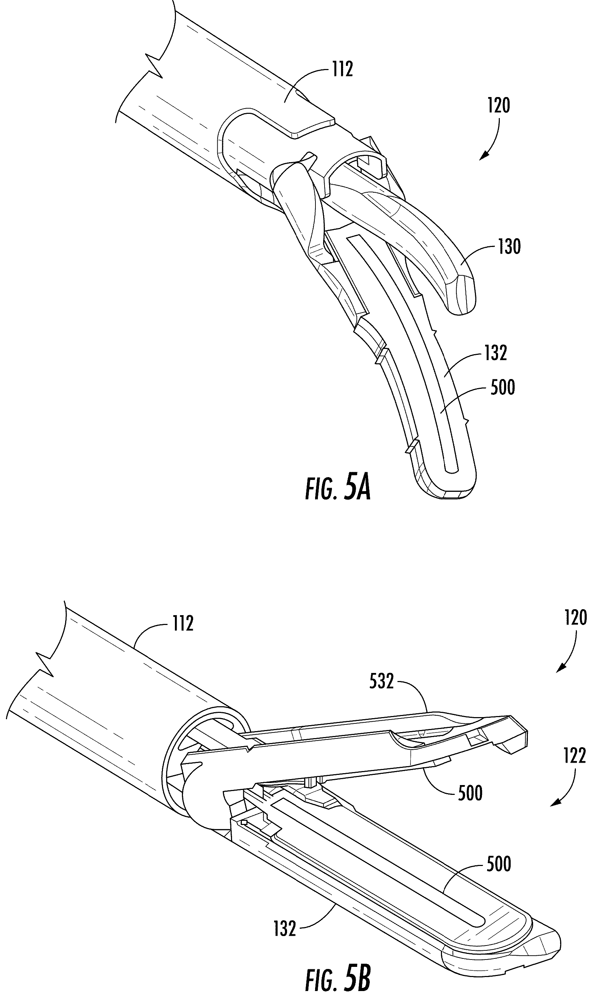

Referring now to FIGS. 1 and 2, in an illustrative embodiment, a system 100 for performing an energy-based surgical procedure includes a surgical instrument 102, a transducer 104, and a generator 106. The surgical instrument 102 is illustratively embodied as an ultrasonic surgical instrument, but may be embodied as an electro-surgical surgical instrument or a multi-modal, ultrasonic/elector-surgical surgical instrument in other embodiments. In use, the surgical instrument 102 is usable to perform various surgical procedures including laparoscopic, endoscopic, or traditional open surgical procedures. In doing so, a surgeon may selectively activate an ultrasonic mode (and/or an electro-surgical/RF mode) of the surgical instrument 102. In the ultrasonic mode, the generator 106 drives the transducer 104 to cause an ultrasonic blade 130 of a jaw assembly 122 of an end effector 120 of the surgical instrument 102 to vibrate at a reference frequency, which facilitates the contemporaneous cutting and hemostatic sealing of patient tissue. Additionally or alternatively, in some embodiments, the surgeon may selectively activate an electro-surgical mode of the surgical instrument 102 to deliver an amount of therapeutic RF energy to the patient tissue to effect hemostatic sealing. In such embodiments, the blade 130 may be embodied as an ultrasonic blade 130 or as a mechanical blade designed to cut tissue using mechanical force (e.g., in those embodiments not employing ultrasonic technologies). Furthermore, in some embodiments, the surgical instrument 102 may be configured with only an electro-surgical/RF mode and, in such embodiments, the jaw assembly 122 of the end effector 120 may not include the ultrasonic blade 130 as discussed in more detail below in regard to FIG. 5B.

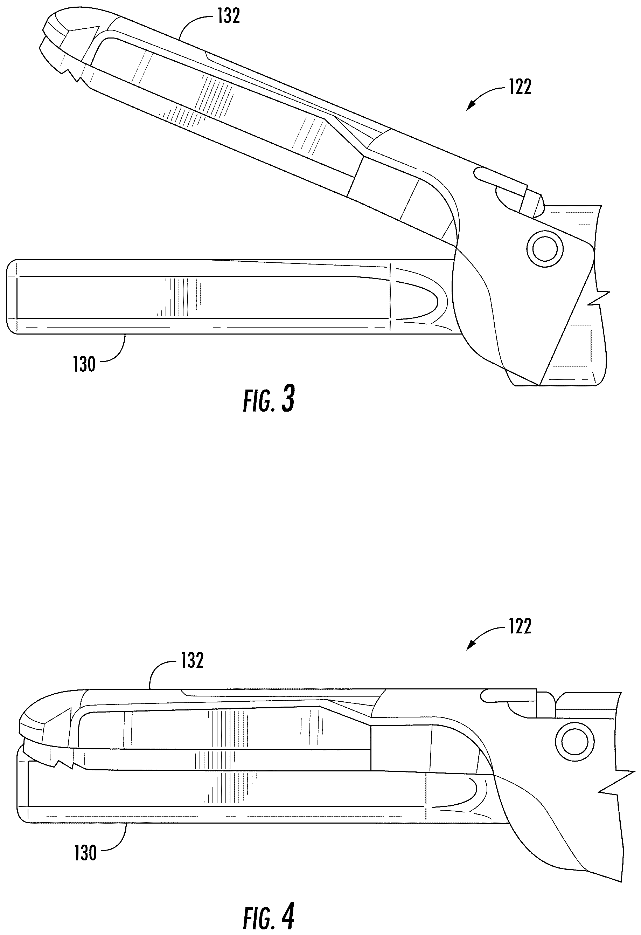

The surgical instrument 102 is illustratively embodied as ultrasonic surgical shears but may be embodied as other types of surgical instruments having an ultrasonic mode and/or electro-surgical mode in other embodiments. In the illustrative embodiment, the surgical instrument 102 includes a handle assembly 110 and an elongated shaft assembly 112, which extends distally away from the handle assembly 110 and may be removably attached to the handle assembly 110 in some embodiments. The elongated shaft assembly 112 includes the end effector 120 located at a distal end opposite the handle assembly 110. The end effector 120 includes the jaw assembly 122, which illustratively includes the ultrasonic blade 130 and a corresponding jaw clamp 132 (but may include two jaw clamps in those embodiments having only an electro-surgical/RF mode). As shown in FIGS. 3 and 4, the jaw assembly 122 is movable between an open state (FIG. 3) in which the jaw clamp 132 is positioned away from the ultrasonic blade 130 and a closed state (FIG. 4) in which the jaw clamp 132 is positioned near or otherwise contacts the ultrasonic blade 130. Actuation of the jaw assembly 122 from the open state to the closed state allows for the grasping, cutting, and coagulation of vessels and/or tissue by the jaw assembly 122. It should be appreciated that the open state may correspond to a degree of openness that is less than a fully opened position of the jaw assembly 122 and the closed state may correspond to a degree of closeness that is less than a fully closed position. That is, the closed state may, for example correspond to a minimal distance between the distal ends of the jaw clamp 132 and the ultrasonic blade 130 and the open state may correspond to a maximum distance between the distal ends of the jaw clamp 132 and the ultrasonic blade 130. However, in other embodiments, the open state may correspond to a fully opened position of the jaw assembly 122 and the closed state may correspond to a fully closed position of the jaw assembly 122.

In those embodiments in which the surgical instrument 102 includes both a ultrasonic mode and an electro-surgical/RF mode, the end effector 120 may include one or more RF electrodes 500 incorporated into the jaw clamp 132 as shown in FIG. 5A. Although the illustrative end effector 120 includes only a single electrode 500 in the embodiment of FIG. 5A, it should be appreciated that the end effector 120 may include additional electrodes 500 in other embodiments (e.g., multiple pads of electrodes 500). The electrode(s) 500 may be embodied as an active electrode configured to the RF energy or as a return electrode configured to "sink" an applied RF energy. In those embodiments utilizing bi-polar RF implementation, the ultrasonic blade 130 may embody the active or return electrode, with the electrode 500 embodying the other active or return electrode. Alternatively, other active or return electrodes may be incorporated on the ultrasonic blade 130 or in another part of the jaw assembly 122 of the end effector 120. In mono-polar implementation, the RF electrode(s) 500 may be embodied as an active electrode, and a return electrode may be attached to a portion of the patient's body.

In those embodiments in which the surgical instrument 102 includes only an electro-surgical/RF mode, the jaw assembly 122 of the end effector 120 includes a jaw clamp 532 in place of the ultrasonic blade 130 as shown in FIG. 5B. In such embodiments, an electrode 500 may be attached to or otherwise incorporated into each jaw clamp 132, 532 and be embodied as an active or a return electrode to facilitate the application of RF energy to tissue captured between the jaw clamps 132, 532. In such embodiments, the surgical instrument 102 may include a knife incorporated into the elongated shaft assembly 112 that is configured to eject outwardly to cut the patient's tissue after sealing of the tissue by the RF energy.

Referring back to FIGS. 1 and 2, in those embodiments including ultrasonic capabilities, the handle assembly 110 includes a receptacle 140 configured to receive the transducer 104 to facilitate connection of the transducer 104 to the handle assembly 110 and the elongated shaft assembly 112. The handle assembly 110 also includes a trigger assembly 150, which includes a primary trigger 152 and a switch assembly 154. The primary trigger 152 is operable by the surgeon to move the jaw assembly 122 of the end effector 120 between the open and closed states. The switch assembly 154 includes one or more buttons, which are selectable by the surgeon to activate (and configure, in some embodiments) the ultrasonic mode and/or the electro-surgical mode of the surgical instrument 102.

The transducer 104 is illustratively connected to the generator 106 by a cable assembly 108. As discussed above, the generator 106 is configured to drive the transducer 104 at a reference or resonant frequency to thereby cause the ultrasonic blade 130 to vibrate. For example, in an illustrative embodiment, the generator 106 may supply an electrical signal to the transducer 104 to cause the ultrasonic blade 130 of the jaw assembly 122 to vibrate longitudinally in the range of, for example, approximately 20 kHz to 250 kHz. In particular embodiments, for example, the ultrasonic blade 130 may vibrate in the range of about 54 kHz to 56 kHz (e.g., at about 55.5 kHz). In other embodiments, the ultrasonic blade 130 may vibrate at other frequencies including, for example, about 31 kHz or about 80 kHz. The excursion of the vibrations at the ultrasonic blade 130 can be controlled by, for example, controlling the amplitude of the electrical signal applied to the transducer 104 by the generator 106. The generator 106 may be activated so that electrical energy may be continuously or intermittently supplied to the transducer 104. The generator 106 also has a power line (not shown) for insertion in an electro-surgical unit or conventional electrical outlet. Additionally or alternatively, the generator 106 may be powered by a direct current (DC) source, such as a battery.

In some embodiments, the generator 106 may be configured to operate in different modes. In such embodiments, the generator 106 may include an ultrasonic generator module 162 for controlling an ultrasonic mode, an electro-surgical/Radio Frequency (RF) generator module 164 for controlling an electro-surgical mode, and/or other generator modules (e.g., a heat generator module) for controlling other operation modes. The various modes of the generator 106 may be operated independently of each other in some embodiments. For example, the generator 106 may activate the ultrasonic mode of the ultrasonic generator module 162 to apply ultrasonic energy to the jaw assembly 122 and subsequently, either therapeutic or sub-therapeutic RF energy may be applied to the jaw assembly 122 by the electro-surgical generator module 164. Alternatively, the activation modes of the generator 106 may be operated simultaneously or contemporaneously with each other.

In the electro-surgical mode, the electro-surgical generator module 164 is configured to generate RF energy at a frequency in the range of about 100 kilohertz (100 kHz) to about 1 megahertz (1 MHz). The generated RF energy is supplied to the patient's tissue via the electrodes 500 of the end effector 120 as described above in regard to FIG. 5. In some embodiments, the electro-surgical generator module 164 may also be configured to selectively provide the RF energy at sub-therapeutic levels to perform various electrical measurements of the patient's tissue. For example, the electro-surgical generator module 164 may be configured to measure an impedance of the patient's tissue using the electrodes 500 and a suitable RF energy level.

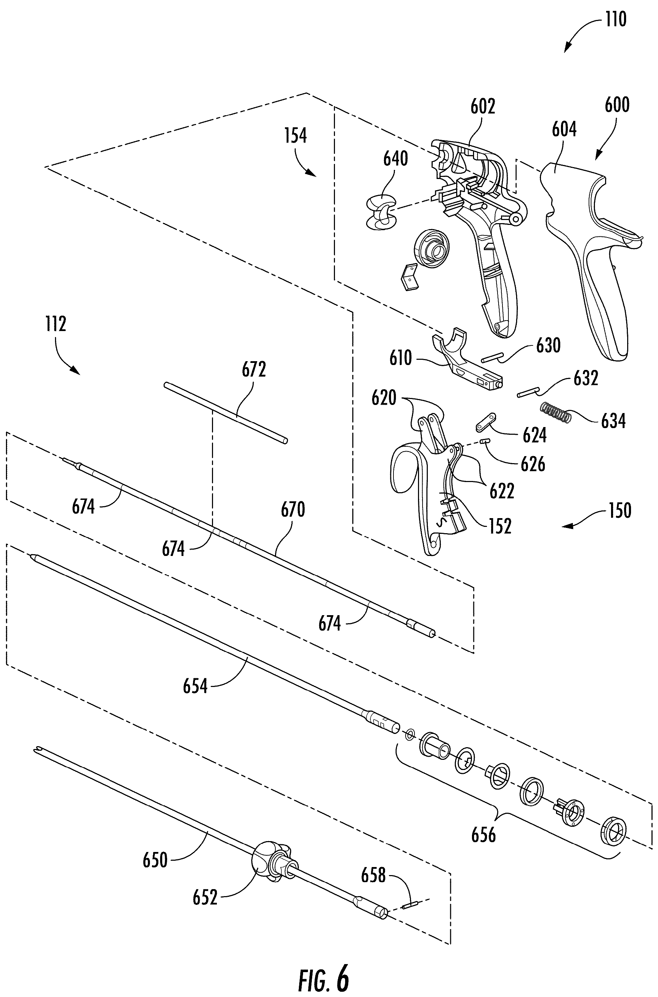

Referring now to FIG. 6, as discussed above, the illustrative surgical instrument 102 includes the handle assembly 110 and the elongated shaft assembly 112, which extends distally away from the handle assembly 110. The handle assembly 110 includes a housing 600, which includes a right half-housing 602 and a left half-housing 604. The half-housings 602, 604 are configured to mate with each other to form the housing 600. To facilitate such mating, each of the half-housings 602, 604 may include various interfaces sized to mechanically align and engage one another to form the housing 600 and enclose the internal working components of the surgical instrument 102.

The primary trigger 152 of the trigger assembly 150 is coupled to a linkage mechanism to translate the rotational motion of the primary trigger 152 to axial motion of a yoke 610, which in turn is configured to move the jaw assembly 122 of the end effector 120 between the open and closed states via the elongated shaft assembly 112. The primary trigger 152 includes a first set of flanges 620 having openings formed therein to receive a first yoke pin 630, which extends through the yoke 610. The primary trigger 152 also includes a second set of flanges 622 configured to receive a first end of a link 624. A trigger pin 626 is received in openings formed in the first end of the link 624 and the second set of flanges 622. The trigger pin 626 forms a trigger pivot point for the primary trigger 152. A second end of the link 624, opposite the first end, is received in a slot formed in a proximal end of the yoke 610 and retained therein by a second yoke pin 632. As the primary trigger 152 is rotated about the pivot point formed from the trigger pin 626, the yoke 610 translates horizontally. A spring 634 is used to bias the yoke forward such that the jaw assembly 122 of the end effector 120 is biased to the open state (or a fully opened state).

As discussed above, the trigger assembly 150 also includes a switch assembly 154. The switch assembly 154 illustratively includes a toggle switch 640, which is selectable to activate one or more switches 642. Activation of the switches 642 electrically energizes an electrical element 644, which electrically energizes the ultrasonic transducer 104 to engage the ultrasonic mode of the surgical instrument 102.

The elongated shaft assembly 112 includes an outer tubular sheath 650 and a rotation knob 652 coupled to the outer cylindrical sheath 650. The rotation knob 652 is operable to rotate the outer cylindrical sheath 650 about an axis defined by the outer cylindrical sheath 650. A reciprocating tubular actuator 654 is located within the outer tubular sheath 650 and mechanically engaged with the end effector 120 on a distal end. The reciprocating tubular actuator 654 is also mechanically engaged, on a proximal end, with the yoke 610 within the handle assembly 110 via coupling elements 656. In embodiments including an ultrasonic mode, an ultrasonic waveguide 670 is located within the reciprocating tubular actuator 654. A distal end of the ultrasonic waveguide 670 is acoustically coupled (e.g., directly or indirectly mechanically coupled) to the ultrasonic blade 130, and a proximal end is acoustically coupled to the transducer 104. The ultrasonic waveguide 670 may be isolated from other components of the elongated shaft assembly 112 by a protective sheath 672 and a number of isolation elements 674. The outer tubular sheath 650, the reciprocating tubular actuator 654, and the ultrasonic waveguide 670 are mechanically engaged together via a pin 658.

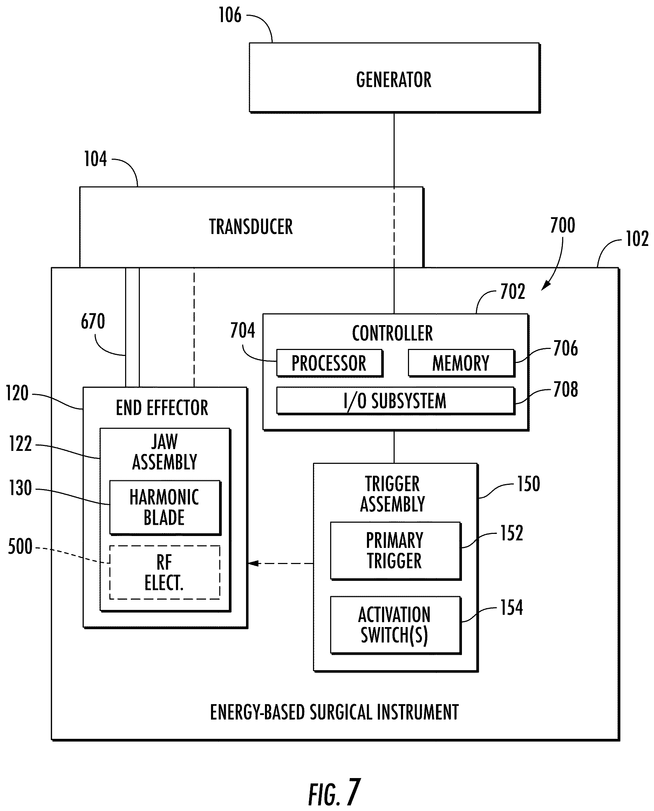

Referring now to FIG. 7, in the illustrative embodiment, the surgical instrument 102 includes a control circuit 700. The control circuit 700 includes a controller 702 and the trigger assembly 150, which cooperate to provide ultrasonic energy to the harmonic blade 130 of the jaw assembly 122 of the end effector 120 and/or RF energy to the RF electrodes 500 of the jaw assembly 122, depending on the operation modes of the surgical instrument 102 as discussed above. In other embodiments, however, the control circuit 700 may include additional or other electronic devices and/or circuit.

The controller 702 may be embodied as any type of controller, functional block, digital logic, or other component, device, circuitry, or collection thereof capable of performing the functions described herein. In illustrative embodiment, the controller 702 includes a processor 704, a memory 706, and an input/output (I/O) subsystem 708. The processor 704 may be embodied as any type of processor capable of performing the functions described herein. For example, the processor 704 may be embodied as a single or multi-core processor(s), digital signal processor, microcontroller, or other processor or processing/controlling circuit. Similarly, the memory 706 may be embodied as any type of volatile and/or non-volatile memory or data storage capable of performing the functions described herein. In operation, the memory 706 may store various data and software used during operation of the control circuit 700 such as executable firmware or software, programs, libraries, and drivers, which may be executed or otherwise used by the processor 704.

The processor 704 and memory 706 are communicatively coupled to other components of the control circuit 700 via the I/O subsystem 708, which may be embodied as circuitry and/or components to facilitate input/output operations between the controller 702 (e.g., the processor 704 and the memory 706) and the other components of the control circuit 700. For example, the I/O subsystem 708 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 708 may form a portion of a system-on-a-chip (SoC) and be incorporated, along with the processor 704 and the memory 706, and other components of the surgical instrument 102, on a single integrated circuit chip. Additionally, in some embodiments, the memory 706, or portions of the memory 706, may be incorporated into the processor 704.

During operation, as discussed above, the controller 702 is configured to control activation of an ultrasonic mode and/or an electro-surgical/RF mode of the surgical instrument 102. To do so, the controller 702 may monitor for activation of the primary trigger 152 and/or one or more activation switches 154 of the trigger assembly 150. In response to activation of the appropriate trigger 152 or switch 154, the controller 702 controls the transducer 104 to generate the ultrasonic energy, which is propagated to the harmonic blade 130 via the ultrasonic waveguide 670. Additionally or alternatively, in response to activation of a corresponding switch 154 of the trigger assembly 150, the controller 702 may be configured to supply an amount of RF energy, via the electro-surgical generator module 164 to the RF electrodes 500 via interconnections 710. It should be appreciated that, although the transducer 104 and the generator 106 are shown as separate components from the energy-based surgical instrument 102 in FIGS. 1 and 7, the transducer 104 and/or the generator 106 may be incorporated into the surgical instrument 102 in other embodiments.

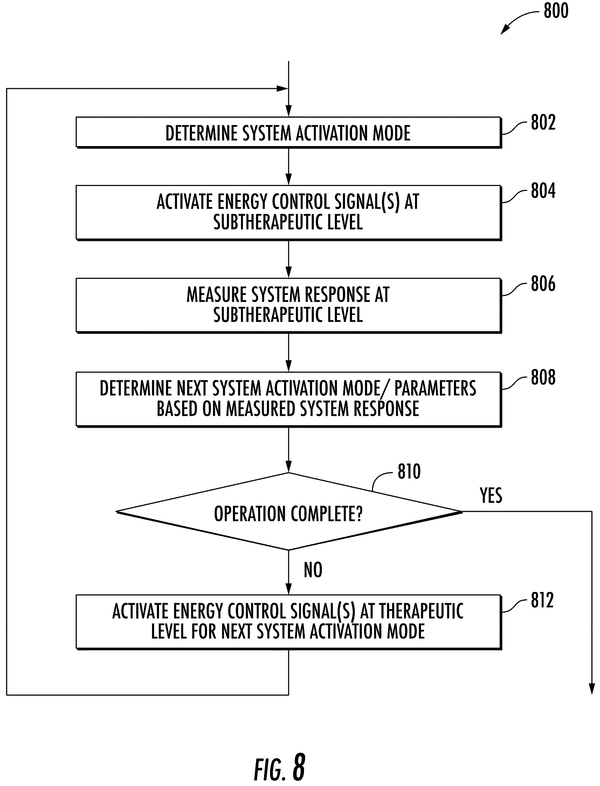

Referring now to FIG. 8, a method 800 for controlling an energy-based surgical instrument 102 is shown. The method 800 may be executed by the controller 702, the generator 106, and/or one or more other microcontrollers or other control elements of the system 100. The method 800 begins in block 802, in which the control element determines a system activation mode for the surgical instrument 102. The system activation mode may include an energy modality (e.g., RF, ultrasound, combined RF and ultrasound, etc.), a surgical operation or firing to be performed with the surgical instrument 102 (e.g., seal, transect, seal and transect, etc.), and/or a sub-operation or phase (e.g., heating, sensing, sealing, cutting, etc.).

In block 804, the control element activates one or more energy control signals at a subtherapeutic level. The subtherapeutic level may be a lower power or energy level that does not cause coagulation, transection, or other therapeutic actions in tissue. The subtherapeutic level may cause other responses in the tissue, such as subtherapeutic heating. The subtherapeutic control may activate ultrasound energy, RF energy, or combined ultrasound and RF energy at the subtherapeutic level.

In block 806, the control element measures a system response at the subtherapeutic level. For example, the control element may measure tissue impedance, acoustic impedance, frequency shift, phase shift, or other responses to application of the subtherapeutic signal.

In block 808, the control element determines a next system activation mode and/or parameters based on the measured system response. For example, the control element may determine whether to switch energy modalities (e.g., from RF to ultrasound, from ultrasound to RF, from a single modality to a combined modality, or other change in energy modality). As another example, the control element may determine whether to change sub-operation or phase, e.g., from pre-heating to sealing, from sealing to transecting, or other change in sub-operation. As another example, the control element may determine one or more parameters for application of therapeutic levels of energy, such as setpoint, amplitude, frequency, crest factor (CF), or other parameters. As yet another example, the control element may determine that the surgical operation (e.g., sealing and/or transecting tissue) has been completed.

In block 810, the control element checks whether the present surgical operation or firing has been completed. If so, the method 800 is completed. The method 800 may be executed again in response to subsequent surgical firings. If the surgical operation is not complete, the method 800 advances to block 812.

In block 812, the control element activates one or more energy control signals at a therapeutic level for the next system activation mode determined as described above. For example, the control element may activate ultrasound and/or RF energy at a setpoint determined as described above or otherwise cause activation of the surgical instrument 102. After activation, the method 800 may loop back to block 802 to continue performing subtherapeutic measurement and control of therapeutic energy application.

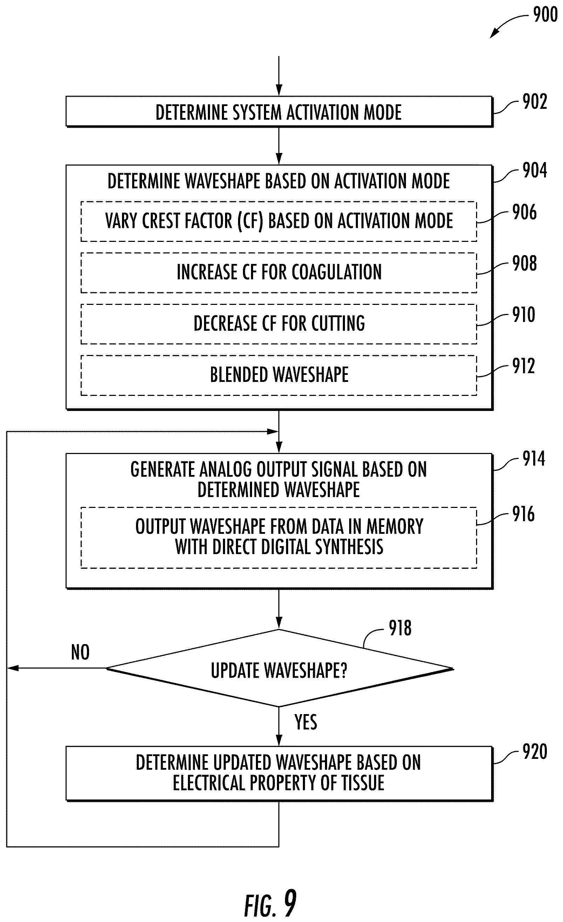

Additionally or alternatively, in some embodiments the control element may perform the operations of the method 800 in a different order and/or in a different combination. Further, in some embodiments the control element may perform additional or different operations and/or make additional or different measurements. Illustrative examples of control operations that may be performed in connection with the surgical instrument 102 are described further below in connection with FIGS. 9-20.

Referring now to FIG. 9, a method 900 for controlling an energy-based surgical instrument 102 is shown. The method 900 may be executed by the controller 702, the generator 106, and/or one or more other microcontrollers or other control elements of the system 100. The method 900 may be executed, for example, in connection with monopolar or bipolar electrosurgery using the surgical instrument 102. The method 900 begins in block 902, in which the control element determines a system activation mode for the surgical instrument 102. The system activation mode may include a surgical operation or firing to be performed with the surgical instrument 102 (e.g., seal, transect, seal and transect, etc.) in a radio frequency (RF) energy mode. The activation mode may include, for example, cutting, coagulation, or blended cutting and coagulation.

In block 904, the control element determines a waveshape for RF energy based on the selected activation mode of the surgical instrument. As described below, the control element may program the generator 106 with the determined waveshape, for example by storing numeric values or other data representing the determined waveshape into a memory of the generator 106.

In some embodiments, in block 906 the control element may vary a crest factor (CF) of the RF signal based on the selected activation mode. Crest Factor (CF) may be defined as the ratio of the instantaneous peak amplitude of a waveform, to its root mean square (RMS) value. The peak amplitude refers to the instantaneous peak voltage that may be required by a load, whereas the RMS is the average load voltage under normal conditions.

Accordingly, the CF specifies the properties of an electrical system such as the purity of a signal or waveform, and the capability of a system such as a power supply to output a particular current or voltage. The ratio is also referred to as peak-to-RMS ratio and is given by:

$$\text{Crest Factor (CF)} = (\text{Vpeak-to-Vrms ratio}) = (\text{Vpeak value})/ (\text{Vrms value})$$

The CF indicates the extreme peaks of a waveform. For a purely DC system with a resistive load, the value should be 1:1 which is also the minimum. A pure sine wave has a crest factor of $\sqrt{2} \approx 1.414$. Accordingly, in some embodiments the waveshape may have a default CF of about 1.4, corresponding to a sinusoidal waveform.

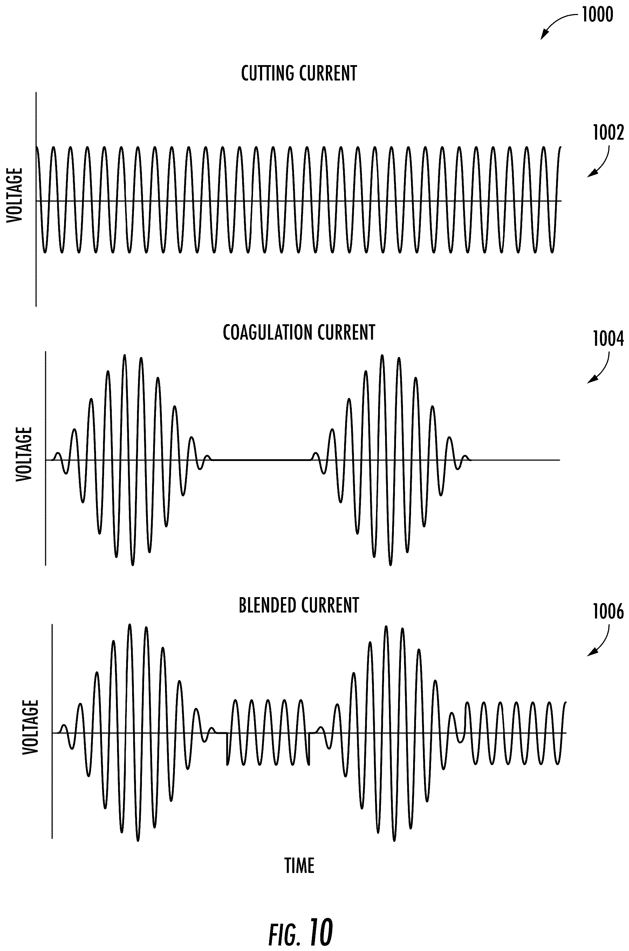

In some embodiments, in block 908 the control element may increase the CF for coagulation. The coagulation waveshape may be characterized by extensive wave modulation, which produces intermittent bursts of damped sine waves of high peak voltages (see, e.g., FIG. 10). These peak voltages result in high tissue temperatures, and hence significant thermal destruction, making this type of waveshape particularly suited for the coagulation of bleeding vessels. Due to its high peak voltage and periods of zero output, this waveshape inherently has the highest CF and therefor may perform best in achieving coagulation.

In some embodiments, in block 910, the control element may decrease the CF for cutting as compared to the CF for coagulation. For example, in the illustrative embodiment the cutting waveshape uses a pure, non-modulated sinusoidal waveform (see FIG. 10), with a CF of about $\sqrt{2} \approx 1.414$. This waveform achieves a highest average power when compared with any other alternating waveform of equal peak voltage, allowing the voltage to be limited when compared with coagulation voltage. The high average power creates a higher current density than is allowed by other waveforms, facilitating a smooth cutting action without extensive thermal damage.

In some embodiments, in block 912 the control element may generate a waveform blended between coagulation and cutting waveforms. Blended waveshapes allow the surgeon to cleanly divide tissue while maintaining a variable degree of hemostasis, depending on the amount of coagulating waveshape used. One method by which blended waveshapes can be created is by modulating a second, lower frequency, higher amplitude sine wave with the sine wave from the cutting generator, producing a higher peak-to-peak voltage. The newly constructed waveform is then delivered in intermittent bursts at a rate determined by the settings of the electrosurgical generator (see, e.g., FIG. 10). This burst effect, although delivered at higher peak-to-peak voltage, contains a lower average power than a pure sinusoidal waveform because of the duty cycle. Due to its high peak voltage and periods of non-zero output, this waveshape inherently has a CF that is in between the CF of a pure sine wave and the CF achieved by the coagulation waveshape. Hence the waveform may produce a "blended" effect of cutting while maintaining a certain level of coagulation.

By controlling CF as described above, tissue effects can be controlled to balance between cutting and coagulation to achieve desired tissue effects while minimizing unintended thermal damage. Traditionally, the therapeutic output waveshape has been a continuous sine wave, having a CF of about 1.4 (including small variations due to amplifier distortion and other signal impurities). The illustrative technologies may dynamically alter waveshapes having CF in the range of about 1.4 to much larger values, for example up to 6, 7, or 9. Accordingly, the described technologies may improve vessel sealing while also reducing transaction times.

In block 914, the control element and/or the generator 106 generates an analog output signal based on the determined waveshape. In some embodiments, in block 916 the waveshape may be output from data in memory using direct digital synthesis. In an illustrative embodiment, a block of 2048 addresses in the generator 106 memory are loaded with 2048 numerical values, each representing a unique point on the desired waveshape. Another mechanism in the generator continuously scans the addresses of that memory block, fetches each of the 2048 numerical values stored in the memory, one at a time, and feeds these also one at a time to a Digital to Analog Converter (DAC). In turn, the DAC converts each of the values to a voltage level which corresponds to each of the mentioned numerical values. In this way the desired waveshape is synthesized. Note that to achieve desired frequency of the output, the complete memory block of 2048 numerical values are scanned at the time period "t" of the desired output frequency "f", where:

$$t = 1/f$$

As described above, the waveshape generated for monopolar or bipolar electrosurgery may be sinusoidal and/or may have other waveshapes. Additionally or alternatively, the waveshape may be altered dynamically during activations or otherwise updated. In block 918, the control element determines whether to update the waveshape. If not, the method 900 loops back to block 914 to continue generating the selected waveshape. If the control element determines to update the waveshape, the method 900 advances to block 920, in which the control element determines an updated waveshape based on one or more electrical properties of the tissue between the jaws of the end effector. For example, in an embodiment, each of the memory addresses in the block may be initially loaded with values representing a sinusoidal waveshape having CF of about 1.4. The values of the memory block may be modified dynamically, for example to increase the CF based on measured electrical properties of the tissue. Measured electrical properties may include, for example, impedance, change in impedance, or other electrical properties. After updating the waveshape, for example by changing values representing the waveshape in memory, the method 900 loops back to block 914 to continue generating the selected waveshape.

Referring now to FIG. 10, diagram 1000 illustrates example waveshapes that may be generated according to the method 900. Curve 1002 represents a cutting waveshape, which is illustratively sinusoidal and thus has a CF of about 1.4 Curve 1004 represents a coagulation waveshape, which illustratively includes bursts of damped sinusoidal signal. Accordingly, the curve 1004 has a higher CF than the curve 1002. Curve 1006 represents a blended cutting/coagulation waveshape, which may be generated by combining aspects of the curves 1002, 1004. For example, the curve 1006 includes bursts of damped sinusoidal signal blended with a continuous sinusoidal signal, and may be generated by modulating a sinusoidal wave with another, lower frequency sinusoidal wave.

In some embodiments, multiple waveshapes such as those shown in FIG. 10 may be used to identify various types of tissue between the jaws of the end effector 120. For example, frequency responses for various types of tissues to various types of waveshapes may be determined empirically. After determining those frequency responses, to perform tissue identification, the control element may program the generator 106 with those various waveshapes in a sequence (e.g., by storing numeric values representing the respective waveshapes into a memory of the generator 106). The control element then applies energy with those respective waveshapes in sequence and measures frequency response of the tissue between the jaws. The control element may compare the measured frequency response of the tissue between the jaws to the predetermined empirical results in order to identify the type of tissue clamped between the jaws.

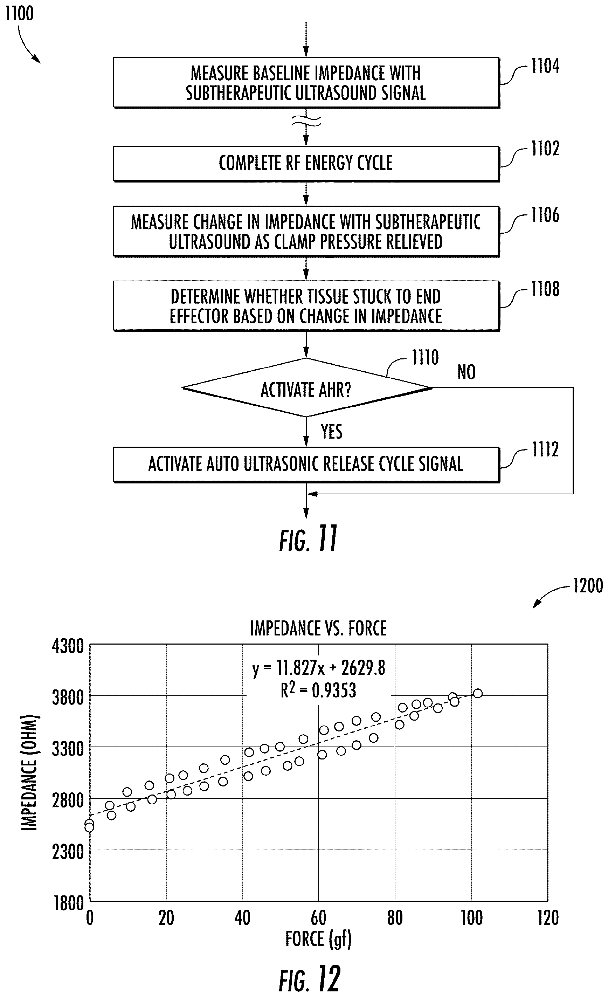

Referring now to FIG. 11, a method 1100 for controlling an energy-based surgical instrument 102 is shown. The method 1100 may be executed by the controller 702, the generator 106, and/or one or more other microcontrollers or other control elements of the system 100. The method 1100 may be executed, for example, in connection with combined RF and ultrasound operation of the surgical instrument 102. The method 1100 begins in block 1102, in which the control element measures baseline acoustic impedance of the surgical instrument 102 with a subtherapeutic ultrasound signal. Subtherapeutic ultrasonic energy, sometimes called subtherapeutic harmonic energy (STHE), corresponds to a current in which the tip displacement of the ultrasonic blade 130 does not produce damage to fully clamped tissue. In some embodiments, a subtherapeutic ultrasonic signal may be sent any time ultrasonic energy is not requested by the user, such as during RF firings and/or during device idle times. Baseline acoustic impedance may be measured, for example, during a device idle time when the jaws of the end effector 120 are empty..

In block 1104, the surgical instrument 102 completes an RF energy cycle (e.g., sealing or sealing and transecting) The RF energy cycle may be performed in response to a user request, and may include bipolar and/or monopolar operation of the surgical instrument 102. The RF energy cycle may be performed, for example, for a predetermined time or until an exit condition has been satisfied.

In block 1106, the control element measures a change in acoustic impedance as clamp pressure in the jaw of the end effector 120 is relieved. Clamp pressure may be relieved, for example in response to the user releasing pressure on the closure handle or otherwise commanding the surgical instrument to release pressure after performance of the RF energy cycle. As described further below, as pressure is relieved from the jaw of the end effector, the acoustic impedance response of the end effector will decrease.

In block 1108, the control element determines whether tissue is stuck to the end effector based on the measured change in impedance. When STHE is used with an ultrasonic instrument 102, there exists a correlation between the forces acting upon the acoustic system and the impedance measured on the acoustic system. For example, the system 100 may exhibit a linear trend of increasing impedance with increasing force (e.g., clamp pressure). The impedance response has been found to be sensitive enough to delineate resulting pressures of a fully clamped state, an open state, and an open state with tissue sticking. In an embodiment, the control element determines whether tissue is stuck to the end effector by comparing the measured acoustic impedance to the baseline acoustic impedance. If impedance is higher than the baseline (e.g., exceeds the baseline by a predetermined threshold or otherwise exceeds the baseline), the control element determines that tissue is stuck to the end effector. Additionally or alternatively, in an embodiment the control element determines whether the tissue is stuck to the end effector by comparing the measured acoustic impedance to the baseline acoustic impedance as described above and by determining whether a closure switch is in an open position. As described above, a full closure switch may be located in the device 102 to tell the generator 106 when the user has fully or mostly compressed the closure handle. The closure switch may move to the "open" position when the user releases pressure on the closure handle. Additionally or alternatively, in an embodiment the control element determines whether the tissue is stuck to the end effector based on an absolute value of the measured acoustic impedance (e.g., by comparing the measured value to a predetermined threshold level or other comparison).

In block 1110, the control element checks whether to activate an automatic ultrasonic release cycle, sometimes called auto-harmonic release (AHR). If the control element determines that tissue is stuck to the jaws, the control element may activate the automatic ultrasonic release cycle. As described above, the control element may compare the measured change in impedance to a calibrated value (e.g., the baseline acoustic impedance) to determine if there is still tissue in the jaws, and if AHR should be activated. Additionally or alternatively, a change in STHE impedance may be calculated and compared after an RF firing and when the closure switch sensor moves to an "open" position. Additionally or alternatively, in some embodiments, the absolute value of STHE impedance may be used to trigger AHR after every RF firing. If the control element determines not to activate AHR, the method 1100 is completed. If the control element determines to activate AHR, the method 1100 advances to block 1112.

In block 1112, the control element activates the automatic ultrasonic release cycle ultrasound signal. The ultrasonic release cycle process provides a relatively small amount of ultrasound energy to the blade 130 in order to reduce tissue sticking to the end effector. After performing the auto ultrasonic release cycle, the method 1100 is completed. The surgical instrument 102 may be used to perform additional surgical operations (e.g., additional cutting or coagulation operations using RF and/or ultrasonic energy). Accordingly, as described above, the method 1100 may prevent performing AHR while there is pressure in the jaws of the end effector, thereby avoiding transecting tissue during the AHR process. Accordingly, the method 1100 may mitigate risk of accidental transection while removing a "time-to-wait" variable and without additional hardware sensors.

Referring now to FIG. 12, chart 1200 illustrates the correlation between the forces acting upon an acoustic system and the acoustic impedance measured on the acoustic system for an illustrative end effector. As shown, in the illustrative embodiment, the system 100 exhibits a linear trend of increasing acoustic impedance with increasing force (e.g., increasing clamp pressure). Other embodiments, even with different loading paths and magnitudes, exhibit a similar linear trend of increasing impedance with increasing force.

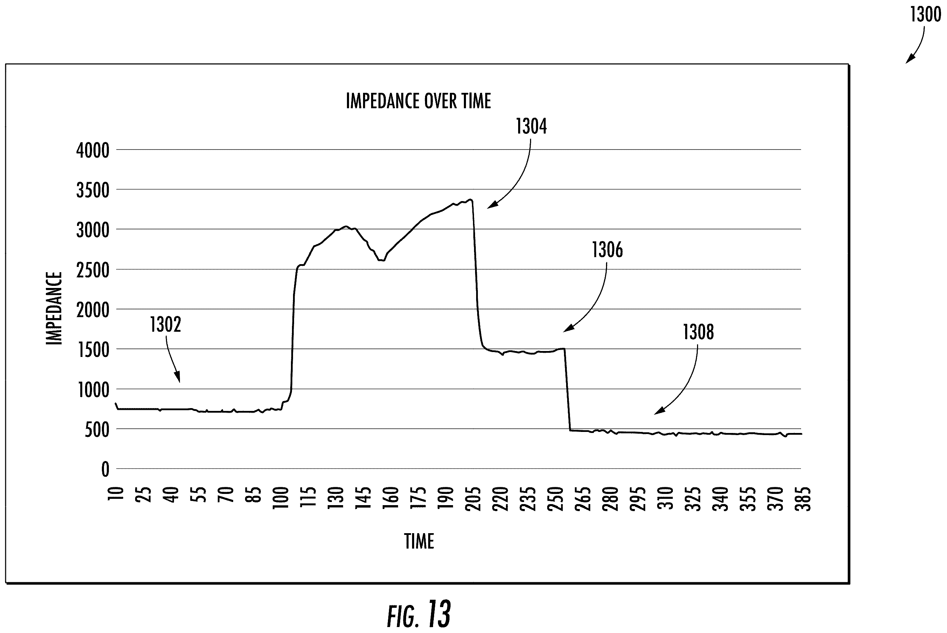

Referring now to FIG. 13, chart 1300 illustrates impedance response of a system 100 measured using subtherapeutic harmonic impedance sensing. The chart 1300 illustrates a base impedance 1302, impedance 1304 with jaws fully closed and tissue in the jaws, impedance 1306 with jaws open with tissue sticking, and impedance 1308 with jaws open in air. As shown, the impedance 1306 with tissue sticking is higher than the baseline impedance 1302.

As described above, the subtherapeutic signal could be incorporated in the triggering of auto-harmonic release (AHR) in multiple scenarios. For example, after the completion of an RF energy cycle, the impedance measured by STHE would be baselined. As pressure is relieved from the jaw of the end effector, the impedance response would decrease. The delta in impedance could be compared to a calibrated value to determine if there is still tissue in the jaws, and if AHR should be activated. As another example, delta in STHE impedance could be calculated and compared after an RF firing AND the closure switch sensor moves to "open." As another example, the absolute value of STHE impedance could be used to trigger AHR after every RF firing.

The following is a non-exhaustive list of embodiments which may or may not be claimed:

1. A method for controlling a surgical instrument, the method comprising:

activating, by a control element, a first energy signal at a subtherapeutic level, wherein the first energy signal is output by a surgical generator coupled to the surgical instrument, and wherein the surgical instrument comprises an end effector configured to deliver energy to tissue of a patient;
measuring, by the control element, a system response in response to activating the first energy signal;
determining, by the control element, an activation parameter based on the system response; and
activating, by the control element, a second energy signal at a therapeutic level according to the activation parameter, wherein the second energy signal is output by the surgical generator.

2. The method of embodiment 1, wherein the first energy signal comprises an ultrasound signal or a radio frequency (RF) signal; and the second energy signal comprises an ultrasound signal or a radio frequency (RF) signal.
3. The method of embodiment 1, wherein the system response comprises impedance, rate of change of impedance, or acoustic impedance.
4. The method of embodiment 1, wherein the activation parameter comprises power, current, or duration.
5. A method for controlling a surgical instrument, the method comprising:

determining, by a control element of the surgical instrument, an activation mode for the surgical instrument, wherein the surgical instrument comprises an end effector having a first electrode configured to deliver radio frequency (RF) energy to tissue of a patient;
determining, by the control element, a waveshape for an energy signal based on the activation mode;
programming, by the control element, an electrosurgical generator coupled to the surgical instrument with the waveshape in response to determining the waveshape; and
delivering, by the electrosurgical generator, the energy signal to the electrode of the end effector in response to programming the electrosurgical generator.

6. The method of embodiment 5, wherein the activation mode comprises a cutting mode, a coagulation mode, or blended mode, and wherein determining the waveshape comprises determining a crest factor for the waveshape based on the activation mode.
7. The method of embodiment 6, wherein determining the waveshape comprises determining a first waveshape having a first crest factor corresponding to a sinusoidal waveshape when the activation mode is the cutting mode.
8. The method of embodiment 7, wherein determining the waveshape comprises determining a second waveshape having a second crest factor larger than the first crest factor when the activation mode is the coagulation mode.
9. The method of embodiment 8, wherein the second waveshape comprises a pulse train of damped sinusoidal waves.
10. The method of embodiment 8, wherein determining the waveshape comprises determining a third waveshape having a third crest factor between the first crest factor and the second crest factor when the activation mode is the blended mode.
11. The method of embodiment 10, wherein the third waveshape comprises a sinusoidal wave modulated with a second, lower frequency sinusoidal wave.
12. The method of embodiment 5, wherein delivering the energy signal comprises generating an analog output signal for the energy signal with direct digital synthesis.
13. The method of embodiment 12, wherein programming comprises storing digital values indicative of the waveshape in a memory of the electrosurgical generator.
14. The method of embodiment 5, further comprising:

determining, by the control element, an updated waveshape for the energy signal based on an electrical property of tissue sensed by the end effector; and
updating, by the control element, programming of the electrosurgical generator with the updated waveshape.

15. The method of embodiment 14, wherein determining the updated waveshape comprises increasing a crest factor of the waveshape from a predetermined crest factor associated with a sinusoidal waveshape.

16. A method for controlling a surgical instrument, the method comprising:

performing, by the surgical instrument, a radio frequency (RF) energy cycle with a first electrode of an end effector of the surgical instrument while pressure is applied in a jaw of the surgical instrument;

relieving pressure in the jaw of the surgical instrument after performing the RF energy cycle;

measuring, by the control element, a first acoustic impedance of the surgical instrument with a subtherapeutic ultrasound signal using an ultrasonic blade of the end effector after relieving the pressure in the jaw of the surgical instrument;

determining, by the control element, whether tissue is stuck to the jaw of surgical instrument based on the first acoustic impedance; and

activating, by the control element, an automatic ultrasonic energy release cycle with the ultrasonic blade of the surgical instrument in response to determining that tissue is stuck to the jaw of the surgical instrument.

17. The method of embodiment 16, further comprising:

measuring, by the control element, a baseline acoustic impedance of the surgical instrument with the subtherapeutic ultrasound signal;

wherein determining whether the tissue is stuck comprises comparing the first acoustic impedance to the baseline acoustic impedance.

18. The method of embodiment 17, wherein determining whether the tissue is stuck further comprises determining whether a closure switch of the surgical instrument moves to an open position.

19. The method of embodiment 16, wherein determining whether the tissue is stuck comprises determining whether the tissue is stuck based on an absolute value of the first acoustic impedance.

20. The method of embodiment 16, further comprising measuring, by the control element, acoustic impedance of the surgical instrument with the subtherapeutic ultrasound signal during the RF energy cycle or during a device idle time.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the spirit of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the spirit and scope of the present disclosure as defined by the appended claims.

**Claims**

1.    A computer-implemented method for controlling a surgical instrument, the method comprising:

activating, by a control element, a first energy signal at a subtherapeutic level, wherein the first energy signal is output by a surgical generator coupled to the surgical instrument, and wherein the surgical instrument comprises an end effector configured to deliver energy to tissue of a patient;

measuring, by the control element, a system response in response to activating the first energy signal, wherein the system response optionally comprises one or more of impedance, rate of change of impedance and acoustic impedance;

determining, by the control element, an activation parameter based on the system response, wherein the activation parameter optionally comprises one or more of power, current and duration; and

activating, by the control element, a second energy signal at a therapeutic level according to the activation parameter, wherein the second energy signal is output by the surgical generator.

2.    A system comprising:

a surgical instrument comprising an end effector configured to deliver energy to tissue of a patient;

a surgical generator coupled to the surgical instrument; and
a control element configured to:

(i) activate a first energy signal at a subtherapeutic level, wherein the first energy signal is output by the surgical generator;
(ii) measure a system response in response to activating the first energy signal, wherein the system response optionally comprises one or more of impedance, rate of change of impedance and acoustic impedance;
(iii) determine an activation parameter based on the system response, wherein the activation parameter optionally comprises one or more of power, current and duration; and
(iv) activate a second energy signal at a therapeutic level according to the activation parameter, wherein the second energy signal is output by the surgical generator.

3. The method of claim 1 or the system of claim 2, wherein the first energy signal comprises an ultrasound signal or a radio frequency (RF) signal; and the second energy signal comprises an ultrasound signal or a radio frequency (RF) signal.

4. A computer-implemented method for controlling a surgical instrument, the method comprising:

determining, by a control element of the surgical instrument, an activation mode for the surgical instrument, wherein the surgical instrument comprises an end effector having a first electrode configured to deliver radio frequency (RF) energy to tissue of a patient;
determining, by the control element, a waveshape for an energy signal based on the activation mode;
programming, by the control element, an electrosurgical generator coupled to the surgical instrument with the waveshape in response to determining the waveshape; and
delivering, by the electrosurgical generator, the energy signal to the electrode of the end effector in response to programming the electrosurgical generator.

5. A system comprising:

a surgical instrument comprising a control element and an end effector having a first electrode configured to deliver radio frequency (RF) energy to tissue of a patient; and
an electrosurgical generator coupled to the surgical instrument;
the control element configured to:

(i) determine an activation mode for the surgical instrument;
(ii) determine a waveshape for an energy signal based on the activation mode; and
(iii) program the electrosurgical generator with the waveshape in response to determining the waveshape; and

the electrosurgical generator configured to:
deliver the energy signal to the electrode of the end effector in response to programming the electrosurgical generator.

6. The method of claim 4 or the system of claim 5, wherein the activation mode comprises a cutting mode, a coagulation mode, or blended mode, and wherein determining the waveshape comprises determining a crest factor for the waveshape based on the activation mode.

7. The method or system of claim 6, wherein determining the waveshape comprises:

(i) determining a first waveshape having a first crest factor corresponding to a sinusoidal waveshape when the activation mode is the cutting mode;
(ii) optionally determining a second waveshape having a second crest factor larger than the first crest factor when the activation mode is the coagulation mode, wherein the second waveshape optionally comprises a pulse train of damped sinusoidal waves; and
(iii) optionally determining a third waveshape having a third crest factor between the first crest factor and the second crest factor when the activation mode is the blended mode, wherein the third waveshape optionally comprises a sinusoidal wave modulated with a second, lower frequency sinusoidal wave.

8. The method of claim 4, the system of claim 5, or the method or system of claims 6 or 7, wherein delivering the energy signal comprises generating an analog output signal for the energy signal with direct digital synthesis.

9. The method or system of any one of claims 4 to 8, wherein programming comprises storing digital values indicative of the waveshape in a memory of the electrosurgical generator.

10. The method of any one of claims 4 to 9, further comprising:

   determining, by the control element, an updated waveshape for the energy signal based on an electrical property of tissue sensed by the end effector; and
   updating, by the control element, programming of the electrosurgical generator with the updated waveshape.

11. The method of claim 10, wherein determining the updated waveshape comprises increasing a crest factor of the waveshape from a predetermined crest factor associated with a sinusoidal waveshape.

12. A computer-implemented method for controlling a surgical instrument, the method comprising:

   performing, by the surgical instrument, a radio frequency (RF) energy cycle with a first electrode of an end effector of the surgical instrument while pressure is applied in a jaw of the surgical instrument;
   relieving pressure in the jaw of the surgical instrument after performing the RF energy cycle;
   measuring, by the control element, a first acoustic impedance of the surgical instrument with a subtherapeutic ultrasound signal using an ultrasonic blade of the end effector after relieving the pressure in the jaw of the surgical instrument;
   determining, by the control element, whether tissue is stuck to the jaw of surgical instrument based on the first acoustic impedance; and
   activating, by the control element, an automatic ultrasonic energy release cycle with the ultrasonic blade of the surgical instrument in response to determining that tissue is stuck to the jaw of the surgical instrument.

13. A system comprising:

   a surgical instrument having an end-effector with a jaw and a first electrode, the surgical instrument configured to perform a radio frequency (RF) energy cycle with the first electrode while pressure is applied in a jaw of the surgical instrument, and to relieve pressure in the jaw of the surgical instrument after performing the RF energy cycle; and
   a control element configured to:

      (i) measure a first acoustic impedance of the surgical instrument with a subtherapeutic ultrasound signal using an ultrasonic blade of the end effector after relieving the pressure in the jaw of the surgical instrument;
      (ii) determine whether tissue is stuck to the jaw of surgical instrument based on the first acoustic impedance; and
      (iii) activate an automatic ultrasonic energy release cycle with the ultrasonic blade of the surgical instrument in response to determining that tissue is stuck to the jaw of the surgical instrument.

14. The method of claim 12 or the system of claim 13, further comprising:

   measuring, by the control element, a baseline acoustic impedance of the surgical instrument with the subtherapeutic ultrasound signal;
   wherein determining whether the tissue is stuck comprises comparing the first acoustic impedance to the baseline acoustic impedance.

15. The method or system of claim 14, wherein determining whether the tissue is stuck further comprises determining whether a closure switch of the surgical instrument moves to an open position.

16. The method or system of any one of claims 12 to 15, wherein determining whether the tissue is stuck comprises determining whether the tissue is stuck based on an absolute value of the first acoustic impedance.

17. The method or system of claim 16, further comprising measuring, by the control element, acoustic impedance of the surgical instrument with the subtherapeutic ultrasound signal during the RF energy cycle or during a device idle time.

FIG. 1

EP 4 767 976 A1

EP 4 767 976 A1

FIG. 2

132

122

130

FIG. 3

122

132

130

FIG. 4

FIG. 5A

FIG. 5B

110

602 604 600

154

640

630

610 632

620 624

626

622

634

152 150

112

672

674

670

674

674

654

656

650

652 658

**FIG. 6**

*FIG. 7*

800

DETERMINE SYSTEM ACTIVATION MODE — 802

↓

ACTIVATE ENERGY CONTROL SIGNAL(S) AT SUBTHERAPEUTIC LEVEL — 804

↓

MEASURE SYSTEM RESPONSE AT SUBTHERAPEUTIC LEVEL — 806

↓

DETERMINE NEXT SYSTEM ACTIVATION MODE/ PARAMETERS BASED ON MEASURED SYSTEM RESPONSE — 808

↓

OPERATION COMPLETE? — 810    YES →

NO ↓

ACTIVATE ENERGY CONTROL SIGNAL(S) AT THERAPEUTIC LEVEL FOR NEXT SYSTEM ACTIVATION MODE — 812

*FIG. 8*

FIG. 9

1000

CUTTING CURRENT

1002

VOLTAGE

COAGULATION CURRENT

1004

VOLTAGE

BLENDED CURRENT

1006

VOLTAGE

TIME

*FIG. 10*

1100

MEASURE BASELINE IMPEDANCE WITH SUBTHERAPEUTIC ULTRASOUND SIGNAL — 1104

COMPLETE RF ENERGY CYCLE — 1102

MEASURE CHANGE IN IMPEDANCE WITH SUBTHERAPEUTIC ULTRASOUND AS CLAMP PRESSURE RELIEVED — 1106

DETERMINE WHETHER TISSUE STUCK TO END EFFECTOR BASED ON CHANGE IN IMPEDANCE — 1108

ACTIVATE AHR? — 1110   NO

YES

ACTIVATE AUTO ULTRASONIC RELEASE CYCLE SIGNAL — 1112

FIG. 11

1200

IMPEDANCE VS. FORCE

$y = 11.827x + 2629.8$
$R^2 = 0.9353$

IMPEDANCE (OHM)

FORCE (gf)

FIG. 12

FIG. 13

EP 4 767 976 A1

# EUROPEAN SEARCH REPORT

**Application Number**

EP 26 15 0069

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 854 335 A2 (ETHICON LLC [PR]) 28 July 2021 (2021-07-28) | 1-3 | INV. A61B18/14 |
| A | * paragraphs [0013] - [0178]; claim 1; figures 1-2,36 * | 12-17 | A61B17/32 |
| X | US 2010/168742 A1 (SHIBATA NORIKIYO [JP]) 1 July 2010 (2010-07-01) | 4-11 | ADD. A61B18/12 |
| A | * paragraphs [0042] - [0197]; claim 1; figures 1-4,11 * | 12-17 | |
| A | US 2021/169559 A1 (LI WEI [US] ET AL) 10 June 2021 (2021-06-10) * paragraphs [0042] - [0069]; claims 1,11; figures 1A-1E * | 12-17 | |

**TECHNICAL FIELDS SEARCHED (IPC)**

A61B

The present search report has been drawn up for all claims

| Place of search | Date of completion of the search | Examiner |
|---|---|---|
| Munich | 9 May 2026 | Gentil, Cédric |

## ANNEX TO THE EUROPEAN SEARCH REPORT
## ON EUROPEAN PATENT APPLICATION NO.

EP 26 15 0069

This annex lists the patent family members relating to the patent documents cited in  the above-mentioned European search report.
The members are as contained in the European Patent Office EDP file on
The European Patent Office is in no way liable for these particulars which are merely given for the purpose of information.

09-05-2026

| Patent document cited in search report | | Publication date | Patent family member(s) | | Publication date |
|---|---|---|---|---|---|
| EP 3854335 | A2 | 28-07-2021 | BR | 112022012620 A2 | 06-09-2022 |
| | | | CN | 115209820 A | 18-10-2022 |
| | | | EP | 3854335 A2 | 28-07-2021 |
| | | | JP | 7622067 B2 | 27-01-2025 |
| | | | JP | 2023508531 A | 02-03-2023 |
| | | | US | 2021196364 A1 | 01-07-2021 |
| | | | WO | 2021137028 A2 | 08-07-2021 |
| US 2010168742 | A1 | 01-07-2010 | CN | 102209503 A | 05-10-2011 |
| | | | EP | 2371313 A1 | 05-10-2011 |
| | | | JP | 4649545 B2 | 09-03-2011 |
| | | | JP | WO2010076869 A1 | 21-06-2012 |
| | | | US | 2010168742 A1 | 01-07-2010 |
| | | | WO | 2010076869 A1 | 08-07-2010 |
| US 2021169559 | A1 | 10-06-2021 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63740944